# EUROPEAN PATENT SPECIFICATION

(11) **EP 2 738 556 B1**
(45) Date of publication and mention of the grant of the patent: **06.11.2019**
(21) Application number: 12818464.5
(22) Date of filing: 24.07.2012
(51) Int. Cl.: G01N 33/543, G01N 21/64, G01N 21/78, G01N 33/545, G01N 33/566, G01N 33/574

(54) **METHOD FOR MEASURING AMOUNT OF ANALYTE AND DEVICE FOR SPFS**
VERFAHREN ZUM MESSEN DER MENGE EINES ANALYTEN UND VORRICHTUNG FÜR SPFS
PROCÉDÉ DE MESURE DE QUANTITÉ D'UNE SUBSTANCE À ANALYSER ET APPAREIL DE SPFS

(30) Priority: 28.07.2011 JP 2011165498
(43) Date of publication of application: 04.06.2014
(73) Proprietor: Konica Minolta, Inc., Tokyo 100-7015 (JP)
(72) Inventor: IDE, Youichi, Tokyo 100-7015 (JP); KANEKO, Tomonori, Tokyo 100-7015 (JP); NINOMIYA, Hidetaka, Tokyo 100-7015 (JP)
(74) Representative: Gille Hrabal
(86) International application number: PCT/JP2012/068710
(87) International publication number: WO 2013/015282

(56) References cited:
- EP-A1- 1 710 581
- WO-A1-2010/100862
- WO-A1-2010/100862
- WO-A1-2010/123073
- WO-A1-2010/134592
- WO-A1-2010/134592
- JP-A- 4 130 274
- US-A- 5 866 433
- HISASHI NARIMATSU ET AL: "A strategy for discovery of cancer glyco-biomarkers in serum using newly developed technologies for glycoproteomics", FEBS JOURNAL, vol. 277, no. 1, 1 January 2010 (2010-01-01), pages 95-105, XP055082552, ISSN: 1742-464X, DOI: 10.1111/j.1742-4658.2009.07430.x

## Description

### TECHNICAL FIELD

The present invention relates to a method for measuring the amount of an analyte in a sandwich assay using a surface plasmon excitation enhanced fluorescence spectroscopy [SPFS]. More specifically, the present invention relates to a method for measuring the amount of an analyte, in which method a fluorescently labeled lectin is used as a secondary antibody in a sandwich assay using a SPFS, and a device for SPFS which is used for said measurement method.

### BACKGROUND ART

In order for proteins, which are a major factor bearing the biological functions in living bodies, to systematically work in the cellular society, post-translational modifications including glycosylation play a very important role. In recent years, it has been revealed one after another that almost all proteins in living bodies are glycosylated (modified with a sugar chain), and the sugar chains added to proteins play an important role in various scenes in biological phenomena, including viral infection, parasitization of protozoa, infection, toxin binding, hormone binding, fertilization, development and differentiation, protein stability, cancer cell metastasis, apoptosis, and so on.

For analysis of the functions of sugar chains, structural analysis of the sugar chains is first required. It is expected that the importance of structural analysis methods for sugar chains will be increased also in the future. However, structural analysis of sugar chains has required a lot of time, effort and experience. Accordingly, it has been demanded to develop a system which, more simply, at high speed and with a high sensitivity and a high accuracy, can extract the structural features of a variety of sugar chains and identify them from each other, not to pursue complete structure determination based on conventional techniques.

It is known that the binding between a sugar chain and a protein showing interaction with the sugar chain (representatively a lectin) is generally a weak interaction as compared to that of the general dissociation constants of antigen-antibody reaction or the like (K_{D} = 10⁻⁸ or lower). The dissociation constant [K_{D}] therebetween is often 10⁻⁶ M or higher. Further, it is known that the interaction between a sugar chain and a protein showing interaction with the sugar chain consists of relatively fast dissociation and association reactions, and, as a result, the equilibrium therebetween tends to incline to the dissociation side by washing operation or the like, as compared to general interactions between proteins or between complementary nucleotide fragments. For example, also in the case where purification of a lectin by a glycoprotein-immobilized column or the like is carried out, when the binding of the lectin is weak, a phenomenon that the lectin flows out of the column during washing operation is often observed.

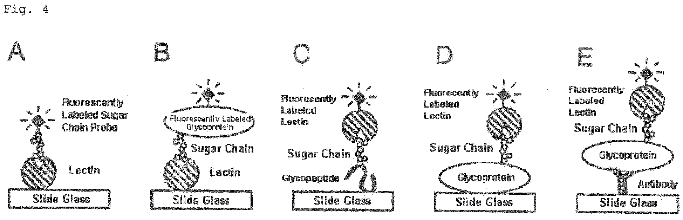

In Patent Document 1, a technique of sugar chain analysis, which is a microarray analysis using the interaction between a lectin and a sugar chain, and in which fluorescence is detected on the basis of evanescent excitation (by a microarray scanner device), is described. Evanescent light (near-field light) is a faint light which, at the time when an excitation light is totally reflected inside a glass, comes out within the area whose height from the interface is 200 to 300 nm (about half of the excitation wavelength), and enables selective observation of a fluorescent substance with which a probe or the like is labeled and which is being captured within said area by the interaction between a lectin and a sugar chain, with almost no excitation of a fluorescent substance with which a probe or the like is labeled and which is in a position far from said area and is moving by Brownian motion (not being captured). As specific modes for such selective observation, a mode in which, as in A and B in Fig. 4 (Fig. 9 of Patent Document 1), a lectin is immobilized on a slide glass and a fluorescently labeled sugar chain probe or a fluorescently labeled glycoprotein is bound thereto; a mode in which, as in E in the same figure, an antibody is immobilized on a slide glass, and a glycoprotein is bound thereto, and then a fluorescently labeled lectin is bound thereto (sandwich assay), and so on are described.

However, there is a problem that, since the amount of the fluorescence that can be excited by evanescent wave (near-field light) is very small, it is needed for a large number of labeled sugar chains to bind to the lectins on the substrate in order to be recognized as a fluorescence signal. Particularly, in the aspect of diagnosis of diseases, since sugar chains that are thought to be a key to diagnose a disease exist in blood only in a very small amount, the detection sensitivity of the measurement method using evanescent wave is not sufficient, and correct diagnosis may be difficult to carry out. In addition, in the technique of Patent Document 1, since the reaction is proceeded in a form of a microarray on which plural lectins are immobilized, a problem that the technique can not provide any sufficient quantitativity also still remains.

The Patent Document 2 discloses a SPFS measurement with binding molecules immobilized on a spacer layer and an analyte-specific fluorescent compound, possibly a labelled lectin, in solution.

The Patent Document 3 discloses a SPFS sensor having binding molecules immobilized inside a three-dimensional polymeric layer.

### PRIOR ART DOCUMENTS

### PATENT DOCUMENTS

[Patent Document 1] WO 2005/064333
[Patent Document 2] US5,866,433
[Patent Document 3] WO2010/134592

### SUMMARY OF THE INVENTION

### PROBLEMS TO BE SOLVED BY THE INVENTION

Conventionally, for analysis of the interaction of a sugar chain with a lectin whose dissociation rate constant is high, a surface plasmon resonance [SPR], which is a technique that is non-labeled (does not use a fluorescent label) and uses a channel(s), including BIACORE, has been used. However, in such analysis, a high-affinity (i.e., binding ability is high) lectin and a high concentration of analyte are needed, and therefore the analytical capability of the technique was insufficient to quantitatively measure a very small amount of sugar chain in blood.

In addition, the technique of sugar chain analysis using near-field light as described in Patent Document 1 is in a microarray form, not in a form using a channel(s), and does not have any quantitativity, and therefore can only be used for profile analysis of plural sugar chains. Among others, in the modes in which a lectin (s) is/are immobilized (A and B), there is also a problem that reaction with a substance(s) other than an analyte of interest (for example, a single glycolipid, glycoprotein, or the like) also occurs. In other words, if a biological component (for example, blood, body fluid, and/or the like) is applied to a lectin-immobilized substrate, then the substance (s) other than the analyte will also adsorb to the immobilized lectin as long as the substance(s) is/are a glycolipid(s) or a glycoprotein(s) having a sugar chain(s) capable of specifically binding to the lectin, and thereby such modes may become measurement systems that significantly lack the sensitivity and the quantitativity.

On the other hand, regarding the mode in which a lectin is not immobilized, but is used as a fluorescently labeled lectin (E), exemplification as a general description has been made, but actual measurement using this mode has not been performed in the Examples, and therefore its measurement capability has not been verified and its practicality has not been supported.

In other words, it can be said that, so far, there has not been a technique which solves the problems of the sensitivity and the non-specific reaction and enables quantitative measurement of a very small amount of sugar chain in blood.

In view of the above, an object of the present invention is to provide a method by which the amount of a very small amount of analyte (in particular, one having a sugar chain) can be measured with a high accuracy and a device which is used for said measurement method.

### MEANS FOR SOLVING THE PROBLEMS

The present inventors have discovered that use of a substance whose dissociation rate constant is high, like a lectin to a sugar chain or the like, as a (fluorescently labeled) secondary antibody, not as an immobilized primary antibody, is not practical in a conventional, non-labeled measurement technique by SPR or the like that is carried out in a channel system or in a measurement technique using non-enhanced near-field light as described in Patent Document 1, because, in these measurement techniques, quantitative measurement is difficult to perform due to its high dissociation constant, its insufficient bonding amount and the like; however, in a SPFS whose sensitivity has become higher due to the use of enhanced near-field light, sufficiently quantitative measurement can be carried out in spite of said high dissociation constant, and real-time measurement can be carried out since a step in which a washing solution is flowed down is not necessarily needed because of an advantage that non-specific adsorption of a ligand probe (i.e., a lectin) is rather suppressed due to said high dissociation rate constant, thereby completing the present invention.

Accordingly, in the method for measuring the amount of an analyte according to the present invention a lectin labeled with a fluorescent dye is used as a secondary antibody in a sandwich assay using a surface plasmon excitation enhanced fluorescence spectroscopy [SPFS].

It is preferable that said assay be carried out within a channel, and the flow rate of the transferring liquid be from 100 µL/min to 10,000 µL/min.

Said analyte is preferably a marker for detecting any of diseases, more preferably a tumor marker.

In the present invention, reaction between said lectin and said analyte is carried out simultaneously with measurement of the amount of the fluorescence by the SPFS.

A solid phase primary antibody to be used in said assay is preferably an antibody that is immobilized in and outside a solid phased layer having a three dimensional structure. And, said solid phased layer preferably comprises a polymer composed of at least one monomer selected from the group consisting of glucose, carboxymethylated glucose, and monomers contained in any of vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones.

The amount of a sample to be supplied to said channel is preferably from 5 µL to 1,000 µL.

In addition, the device for SPFS for use in the present invention is characterized by comprising a plasmon sensor, on one surface of which a ligand is immobilized, wherein an analyte binds to said ligand, and a lectin labeled with a fluorescent dye further binds to the analyte.

In the device for SPFS for use in the present invention, said analyte is preferably a tumor marker, and said ligand is preferably an antibody that is immobilized in and outside a solid phased layer having a three dimensional structure. Said solid phased layer preferably comprises a polymer composed of at least one monomer selected from the group consisting of glucose, carboxymethylated glucose, and monomers contained in any of vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones.

### EFFECTS OF THE INVENTION

The present invention can provide a method for measuring the amount of an analyte, in which method, by using a SPFS, enhanced electric field unrealizable by conventional total-reflection systems can be created, and a very small amount of sugar chain, which is captured by an antibody and "a ligand probe whose dissociation rate constant is high", can be recognized.

In the present invention, use of a lectin, which is "a ligand probe whose dissociation rate constant is high", as a labeled lectin, not as a solid phase lectin, enables recognition of a very small amount of sugar chain in blood without any concern for non-specific reaction of the lectin.

In addition, by carrying out the measurement method of the present invention within a channel, an assay signal and an assay blank can be separated, and the ability to recognize a very small amount of the bound sugar chain is increased. Further, a series of steps can be carried out rapidly and simply.

In a general fluorescence measurement after washing in a SPFS, problems of signal decrease associated with dissociation of captured analyte, difficulty in rapid diagnosis, and so on have occurred. However, in the present invention, a real-time measurement can be carried out, and therefore a highly sensitive and rapid processing is possible.

### BRIEF DESCRIPTION OF THE DRAWINGS

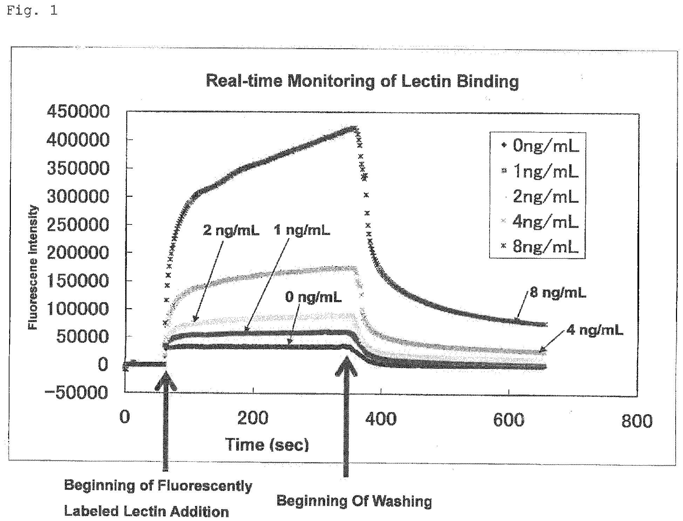

Fig. 1 shows the results of the real-time measurement in Example 1 (1-3), and is a graph obtained by monitoring and detecting the binding reaction of the fluorescently labeled lectin in SPFS in real time, and plotting with respect to each addition concentration of the fluorescently labeled lectin. It was revealed that the reaction amount was increased over time from the beginning of the fluorescently labeled lectin addition until immediately before washing, and rapidly decreased after washing, and it is shown that even a very small amount of binding can be quantified by measuring the reaction amount before washing.

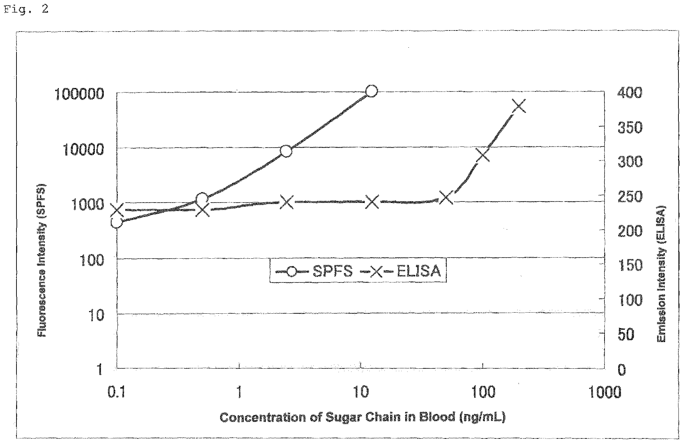

Fig. 2 shows the results of comparison between a conventional method (ELISA; Comparative Example 1) and SPFS (Example 1) in case of using a lectin whose dissociation rate constant is high. It was revealed that, even if a lectin whose dissociation rate constant is high was used and even after washing of the fluorescently labeled lectin, in the SPFS, detection of a very small amount of sugar chain is possible.

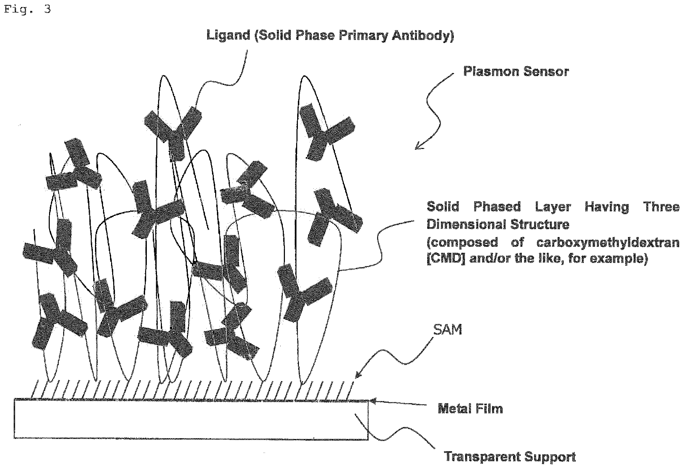

Fig. 3 shows a schematic diagram of the cross section of a plasmon sensor used in the present invention, in which, in a sandwich assay using a surface plasmon excitation enhanced fluorescence spectroscopy [SPFS], the primary antibody (ligand) immobilized on said plasmon sensor is preferably an antibody that is immobilized in and outside a solid phased layer having a three dimensional structure.
Fig. 4 shows Fig. 9 A to E of Patent Document 1. MODE FOR CARRYING OUT THE INVENTION

The method for measuring the amount of an analyte of the present invention and the device for SPFS which is used for said measurement method of the present invention will be specifically described below.

### <Measurement Method>

The method for measuring the amount of an analyte according to the present invention is characterized in that a lectin labeled with a fluorescent dye, preferably said lectin whose dissociation rate constant [k_{d}] is from 1.0 x 10⁻⁶ to 1.0 x 10⁻³ (S⁻¹), is used as a secondary antibody in a sandwich assay using a surface plasmon excitation enhanced fluorescence spectroscopy [SPFS; Surface Plasmon-field enhanced Fluorescence Spectroscopy].

In the present invention, the dissociation rate constant is used also as an index of the stability of complexes, and numerically expresses a ratio of dissociation of complexes per second. It is represented by "k_{d}", and the unit thereof is S⁻¹, i.e., 1/sec. In the present invention, the dissociation rate constant represents a ratio of dissociation of complexes composed of analytes and probes (or ligands). The term "sandwich assay" means, as is conventional, an assay in which an analyte can be detected only by using a solid phase primary antibody (ligand), an analyte and a secondary antibody (probe) in combination, said secondary antibody being labeled with a substance that can be optically detected, such as a fluorescent dye.

In the present invention, as the dissociation rate constant, those that are measured at room temperature by the capture method as described below are used.

The capture method is a method in which a molecule having an affinity to a ligand is immobilized onto a sensor chip or the like. For example, if the ligand has a tag (such as GST, Flag, Fc, or the like), an antibody (a capture molecule) against the tag is immobilized by amine coupling onto "Sensor Chip CM5" (manufactured by Biacore Life Sciences), and next, the ligand is added thereto to be temporary immobilized (captured) by antigen-antibody reaction. Thereafter, an analyte is added thereto to measure their interaction, and is removed together with the ligand for regeneration.

In the present invention, the term "lectin labeled with a fluorescent dye" is also described simply as "labeled lectin", "fluorescently labeled lectin" or "lectin which is fluorescently labeled".

In case where the measurement method of the present invention is carried out within a channel, the present invention enables measurement of the fluorescence amount (the fluorescence intensity) by a SPFS without washing a fluorescently labeled lectin after the reaction between an analyte and the fluorescently labeled lectin, even during and immediately after the reaction between the analyte and the fluorescently labeled lectin. In other words, in the present invention, reaction between an analyte and a fluorescently labeled lectin can be carried out simultaneously with measurement of the amount of the fluorescence by the SPFS. This is because non-specific adsorption is suppressed due to the high dissociation rate constant of the lectin, and, when the fluorescence is detected, the fluorescently labeled lectin that undergoes the binding reaction can be selectively observed with almost no excitation of the fluorescent dye of the fluorescently labeled lectin which is moving by Brownian motion since enhanced electric field by a SPFS is only within the area whose height from the interface is 200 to 300 nm (about half of the excitation wavelength).

In the present invention, when a sandwich assay using a SPFS is carried out, it is preferable to use a plasmon sensor which comprises a transparent support, a metal film formed on one surface of a transparent support, a self-assembled monolayer [SAM] formed on the other surface of said metal film which surface is not in contact with the transparent support, and a ligand immobilized on the other surface of said SAM which surface is not in contact with said metal film. The invention uses a plasmon sensor which comprises, as shown in Fig. 3, a solid phased layer having a three dimensional structure in addition to said transparent support, said metal film, said SAM and said ligand, wherein said solid phased layer is formed on the other surface of said SAM which surface is not in contact with said metal film, and said ligand is immobilized in and outside said solid phased layer.

A measurement method outside the scope of the present invention uses such a plasmon sensor and comprises the following Steps (i) to (iii): Step (i) contacting a sample that contains an analyte having a sugar chain with the plasmon sensor, and thereafter washing components contained in the sample other than said analyte binding to a ligand; Step (ii) contacting a lectin labeled with a fluorescent dye with the plasmon sensor obtained after Step (i) ; and Step (iii) irradiating laser light from the other surface of a transparent support on which surface said metal film is not formed, measuring the amount of the fluorescence emitted from the excited fluorescent dye, and, from the results, calculating the amount of the analyte contained in the sample.

### (Lectin Labeled with Fluorescent Dye)

It is preferable that the lectin to be used in the present invention have a dissociation rate constant [k_{d}] of from 1.0 x 10⁻⁶ to 1.0 x 10⁻³ (S⁻¹), and be labeled with a fluorescent dye.

Examples of the lectin include lectins belonging to various molecular families which are obtained from animals or plants, fungi, bacteria, viruses or the like, i.e., "R-type lectins" which are related to ricin B chain and which are found in the kingdoms of all organisms including bacteria; "calnexin and calreticulin" which are present in the whole eukaryotic organisms and are involved in folding of glycoproteins, "C-type lectins" which are calcium-requiring and which are present widely in multicellular animals and include a large number of representative lectins such as "selectin", "collectin" and the like; "galectins" which are widely distributed in the animal kingdom and exhibit specificity to galactose; "legume lectins" which form a large family in legumes, and "L-type lectins" which have structural similarity to legume lectins and are involved in intracellular transport in animals; "P-type lectins" which have an ability to bind to mannose 6-phosphate and which are involved in intracellular transport of lysosomal enzymes; "annexins" which bind to an acidic sugar chain such as glycosaminoglycan; "I-type lectins" which belong to the immunoglobulin superfamily and include "siglec", and so on.

Examples of other lectins may include AAL (*Aleuria aurantia* lectin), ACA (*Amaranthus caudatus* lectin), BPL (*Bauhinia purpurea* lectin), ConA (*Canavalia ensiformis* lectin), DBA (Horsegram lectin), DSA (*Datura stramonium* lectin), ECA (*Erythrina cristagalli* lectin), EEL (Spindle Tree lectin), GNA (*Galanthus nivalis* lectin), GSL I (*Griffonia simplicifolia* lectin), GSL II (*Griffonia simplicifolia* lectin), HHL (*Hippeastrum hybrid* lectin), jacalin (jackfruit lectin), LBA (Lima bean lectin), LCA (*Lens culinaris* lectin), LEL (*Lycopersicon esculentum* lectin), LTL (*Lotus tetragonolobus* lectin) , MPA (*Maclura pomifera* lectin), NPA (*Narcissus pseudonarcissus* lectin), PHA-E (*Phaseolus vulgaris* lectin), PHA-L (*Phaseolus vulgaris* lectin), PNA (*Arachis hypogaea* lectin), PSA (*Pisum sativum* lectin), PTL-I (*Psophocarpus tetragonolobus* lectin), PTL-II (*Psophocarpus tetragonolobus* lectin), PWM (*Phytolacca americana* lectin) RCA120 (*Ricinus communis* lectin), SBA (soybean lectin), SJA (*Sophora japonica* lectin), SNA (*Sambucus nigra* lectin), SSA (*Sambucus sieboldiana* lectin), STL (*Solanum tuberosum* lectin), TJA-I (*Trichosanthes japonica* lectin), TJA-II (*Trichosanthes japonica* lectin), UDA (Common Stinging Nettle lectin), UEA I (*Ulex europaeus* lectin), VFA (*Vicia faba* lectin), VVA (*Vicia villosa* lectin), WFA (*Wisteria floribunda* lectin), WGA (Wheat germ lectin), and so on.

### (Fluorescent Dye)

In the present invention, the term "fluorescent dye", with which lectin is labeled, is a general term for substances that emit fluorescence as a result of irradiation of a predetermined excitation light or excitation using field effect, and the term "fluorescence" also includes various kinds of emission such as phosphorescence.

The type of the fluorescent dye to be used in the present invention is not particularly limited, as long as the fluorescent dye is not quenched due to light absorption by the metal film. Any of known fluorescent dyes may be used. In general, a fluorescent dye that allows for use of a fluorometer equipped with a filter rather than a monochromometer and has a large Stokes shift which enhances the detection efficiency, is preferable.

Examples of such fluorescent dye include, for example, fluorescent dyes of the fluorescein family (manufactured by Integrated DNA Technologies, Inc.), fluorescent dyes of the polyhalofluorescein family (manufactured by Applied Biosystems Japan Ltd.), fluorescent dyes of the hexachlorofluorescein family (manufactured by Applied Biosystems Japan Ltd.), fluorescent dyes of the coumarin family (manufactured by Invitrogen Japan K.K.), fluorescent dyes of the rhodamine family (manufactured by GE Healthcare Bioscience Co., Ltd.), fluorescent dyes of the cyanine family, fluorescent dyes of the indocarbocyanine family, fluorescent dyes of the oxazine family, fluorescent dyes of the thiazine family, fluorescent dyes of the squaraine family, fluorescent dyes of the chelated lanthanide family, fluorescent dyes of the BODIPY (registered trademark) family (manufactured by Invitrogen Japan K.K.), fluorescent dyes of the naphthalenesulfonic acid family, fluorescent dyes of the pyrene family, fluorescent dyes of the triphenylmethane family, Alexa Fluor (registered trademark) dye series (manufactured by Invitrogen Japan K.K.) and so on. Further, fluorescent dyes as described in U.S. Patent No. 6,406,297, U.S. Patent No. 6,221,604, U.S. Patent No. 5, 994, 063, U.S. Patent No. 5,808,044, U.S. Patent No. 5,880,287, U.S. Patent No. 5,556,959 and U.S. Patent No. 5,135,717 may also be used in the present invention.

The absorption wavelengths (nm) and the emission wavelengths (nm) of representative fluorescent dyes included in these families are shown in Table 1.

**[Table 1]**

| Fluorescent Dye | Family | Absorption Wavelength (nm) | Emission Wavelength (nm) |
|---|---|---|---|
| Aminomethylcoumarin; AMCA | Coumarin | 350 | 450 |
| Cy2 (registered trademark) | Cyanine | 492 | 510 |
| Fluorescein Isothiocyanate; FITC | Fluorescein | 492 | 520 |
| Cy3 (registered trademark) | Indocarbocyanine | 550 | 570 |
| Tetramethyl Rhodamine Isothiocyanate; TRITC | Rhodamine | 550 | 570 |
| Rhodamine Red-X; RRX | | 570 | 590 |
| Texas Red; TR | | 596 | 620 |
| Cy5 (registered trademark) | Cyanine | 650 | 670 |
| Alexa Fluor (registered trademark) 647 | Cyanine | 650 | 665 |

Furthermore, the fluorescent dye is not limited to the organic fluorescent dyes as mentioned above. For example, fluorescent dyes of rare earth complex systems such as Eu and Tb may be used as the fluorescent dye in the present invention. Rare earth complexes are generally characterized in that the wavelength differences between their excitation wavelengths (about from 310 to 340 nm) and their emission wavelengths (approximately 615 nm in case of Eu complex, and approximately 545 nm in case of Tb complex) are large, and that their fluorescence lifetimes are as long as hundreds of microseconds or more. Examples of commercially available fluorescent dyes of rare earth complex systems include ATBTA-Eu³⁺.

In the present invention, in case where the measurement of fluorescence as described below is carried out, it is desirable to use a fluorescent dye whose maximum fluorescence wavelength is within the wavelength region in which light absorption by metal contained in the metal film is small. For example, when gold is used as the metal film, it is desirable to use a fluorescent dye whose maximum fluorescence wavelength is not less than 600 nm, in order to minimize the influence of light absorption by the gold film. Accordingly, in this case, it is especially desirable to use a fluorescent dye that has a maximum fluorescence wavelength in the near infrared region, such as Cy5 or Alexa Fluor (registered trademark) 647. The use of such a fluorescent dye that has a maximum fluorescence wavelength in the near infrared region is useful also in case where blood is used as a sample, from the standpoint that the influence of light absorption by iron derived from hemocyte components in the blood can be minimized. On the other hand, when silver is used as the metal film, it is desirable to use a fluorescent dye whose maximum fluorescence wavelength is not less than 400 nm.

These fluorescent dyes may be used individually, or two or more of them may be used in combination.

Examples of a method for producing a lectin labeled with a fluorescent dye include, for example, a method in which a carboxyl group is first attached to a fluorescent dye, and the carboxyl group is active esterified by using water-soluble carbodiimide [WSC] (such as 1-ethyl-3-(3-dimethylaminopropyl)carbodiimide hydrochloride [EDC]) and N-hydroxysuccinimide [NHS], and then a dehydration reaction between the active esterified carboxyl group and an amino group contained in the lectin is carried out using water-soluble carbodiimide to immobilize; a method in which a lectin and a fluorescent dye that have isothiocyanate and amino groups respectively are reacted to immobilize; a method in which a lectin and a fluorescent dye that have sulfonyl halide and amino groups respectively are reacted to immobilize; a method in which a lectin and a fluorescent dye that have iodoacetamide and thiol groups respectivwly are reacted to immobilize; a method in which a biotinylated fluorescent dye and a streptavidinylated lectin (or a streptavidinylated fluorescent dye and a biotinylated lectin) are reacted to immobilize, and so on.

### (Analyte)

The analyte is not particularly limited and may be a tumor marker, a signal transducer, a hormone, or the like, as long as it is a molecule or a molecular fragment that has a sugar chain and can be specifically recognized by and can specifically bind to a primary antibody, wherein examples of such "a molecule" or "a molecular fragment" include, for example, nucleic acids (DNAs, RNAs, polynucleotides, oligonucleotides, PNAs (peptide nucleic acids) and the like which may be either single-stranded or double-stranded, or nucleosides, nucleotides and modified molecules thereof); proteins (polypeptides, oligopeptides, and the like); amino acids (including modified amino acids); carbohydrates (oligosaccharides, polysaccharides, sugar chains , and the like); lipids; or modified molecules and complexes thereof.

In particular, a large number of tumor markers which have been used as a biomarker are factors related to any sugar chain, and such markers are suitable as the analyte in the present invention. Specific examples thereof include carcinoembryonic antigens such as AFP [a-fetoprotein], glycoproteins such as PSA [prostate-specific antigen], CA19-9 which is known as a carbohydrate antigen, and so on.

### (Sample)

Examples of a sample include, for example, blood (serum and plasma), urine, nasal cavity liquid, saliva, stool, coelomic fluid (spinal fluid, ascitic fluid, pleural fluid, or the like) and so on, and such a sample may be used after appropriate dilution into a desired solvent, buffer solution or the like. Among these samples, blood, serum, plasma, urine, nasal cavity liquid and saliva are preferable.

### (Contact)

Contact between a plasmon sensor and a sample is preferably performed in a mode in which the plasmon sensor and the sample are brought into contact under the conditions that the transferring liquid circulating in a channel contains a sample, and only one side of the plasmon sensor, on which side a ligand is immobilized, is immersed in said transferring liquid.

### (Channel)

The channel is in the shape of a square pipe or a cylindrical pipe (tube) as described above. In an area into which a plasmon sensor is placed and the vicinity thereof, it preferably has a square pipe-shaped structure; and, in an area for delivering a drug solution and the vicinity thereof, it preferably has a cylindrical pipe (tube)-shaped structure.

As materials thereof, a plasmon sensor part or a channel top board is composed of a homopolymer or a copolymer that contains methyl methacrylate, styrene or the like as a raw material; polyolefin such as polyethylene, and/or the like. In a part for delivering a drug solution, a polymer (s), such as silicone rubber, Teflon (registered trademark), polyethylene, polypropylene and/or the like, is/are used.

In a plasmon sensor part, from the standpoint that the efficiency of the contact with a sample is enhanced and the diffusion distance is shortened, it is preferable that the length and width of the section of the channel in the plasmon sensor part be each independently about from 100 nm to 1 mm.

In a small scale lot (laboratory level), as a method for fixing a plasmon sensor in the channel, a method, in which a sheet, having a channel height of 0 . 5 mm and made of polydimethylsiloxane [PDMS], is first bonded by pressure onto the surface of the plasmon sensor on which surface a metal film is formed such that the site on the plasmon sensor on which site a metal film is formed is surrounded by the sheet; next, the sheet made of polydimethylsiloxane [PDMS] and the plasmon sensor are fixed by fasteners such as screws, is preferable.

In a large scale lot (plant level) for which industrial manufacture is carried out, by using, as a method for fixing a plasmon sensor in the channel, a method in which a sensor substrate is formed on, or a sensor substrate which has been separately produced is fixed onto, an integrally molded article made of plastic; onto the surface of its metal film, (preferably a spacer layer composed of a dielectric(s),) a SAM, a solid phased layer and a ligand are immobilized; and thereafter, it is covered with an integrally molded article made of plastic which corresponds to a channel top board, the manufacture can be carried out. If necessary, a prism may also be integrated into the channel.

### (Transferring Liquid)

The transferring liquid is preferably the same as a solvent or a buffer solution that has been used for diluting the sample; and examples thereof include, for example, phosphate buffered saline [PBS], Tris buffered saline [TBS], HEPES buffered saline [HBS], and so on, but not particularly limited thereto.

The temperature at which the transferring liquid is circulated and the time for which the transferring liquid is circulated vary depending on the type of the sample and/or the like, and are not particularly limited, but are usually at 20 to 40°C and for 1 to 60 minutes, preferably at 37°C and for 5 to 15 minutes.

In case where the measurement method of the present invention is thus carried out within a channel, it is preferable that the flow rate of the transferring liquid be from 100 (µL/min) to 10,000 (µL/min). In addition, the amount of a sample to be supplied to said channel is preferably from 5 µL to 1,000 µL. If the flow rate of a transferring liquid and the amount of a sample are each within the above-described ranges, then non-specific reactions of the lectin can be reduced, and therefore these ranges are suitable from the standpoint of ensuring the specific binding between a lectin and an antigen sugar chain.

### <Plasmon Sensor>

As described above, when carrying out the present invention, a plasmon sensor is used, and the plasmon sensor comprises a transparent support, a metal film and a SAM, and a solid phased layer, and a ligand (a primary antibody as described above is preferable).

### (Transparent Support)

In the present invention, as a substrate supporting the structure of a plasmon sensor, a transparent support is used. The reason why a transparent support is used as a sensor substrate in the present invention is because light irradiation to a metal film as described below is carried out through this transparent support.

The material of a transparent support to be used in the present invention is not particularly limited, as long as the object of the present invention is accomplished. For example, the transparent support may be made of glass, or may be made of plastic, such as polycarbonate [PC], cycloolefin polymer [COP] or the like.

Further, the size (length and width) is not particularly limited, as long as the refractive index at the d line (588 nm) [n_{d}] is preferably from 1.40 to 2.20, and the thickness is preferably from 0.01 to 10 mm, more preferably from 0.5 to 5 mm.

For a transparent support made of glass, as commercially available products, "BK7" (Refractive Index [n_{d}] 1.52) and "LaSFN9" (Refractive Index [n_{d}] 1.85) manufactured by SCHOTT Nippon K.K., "K-PSFn3" (Refractive Index [n_{d}] 1.84), "K-LaSFn17" (Refractive Index [n_{d}] 1.88) and "K-LaSFn22" (Refractive Index [n_{d}] 1.90) manufactured by SUMITA OPTICAL GLASS, Inc., and "S-LAL10" (Refractive Index [n_{d}] 1.72) manufactured by OHARA INC. , and so on are preferable from the standpoint of the optical property and the washability.

The surface of the transparent support is preferably washed with acid and/or plasma, before a metal film is formed on it. The washing treatment with acid is preferably immersion in 0.001 to 1N hydrochloric acid for 1 to 3 hours. Examples of the washing treatment with plasma include, for example, a method of immersion in Plasma Dry Cleaner ("PDC200" manufactured by Yamato Scientific Co., Ltd.) for 0.1 to 30 minutes.

### (Metal Film)

In the plasmon sensor for use in the present invention, a metal film is formed on one surface of said transparent support. This metal film has a role of allowing surface plasmon excitation by irradiation light from light source, generating electric field, and producing emission of a fluorescent dye.

The metal film formed on one surface of the transparent support is preferably composed of at least one metal selected from the group consisting of gold, silver, aluminum, copper and platinum, more preferably composed of gold. These metals may be in a form of alloy thereof. Such metal species are suitable, since they are stable to oxidization and electric field enhancement by surface plasmon becomes high.

When a substrate made of glass is used as a transparent support, in order to more strongly adhere the glass and the metal film as mentioned above, it is preferable to form a metal film of chromium, nickel chromium alloy, or titanium in advance.

Examples of a method by which a metal film is formed on a transparent support include, for example, a sputtering method, a vapor deposition method (such as a resistance heating vapor deposition method, an electron beam vapor deposition method and the like), an electrolytic plating method, an electroless plating method, and so on. It is preferable to form a film of chromium and/or a metal film by a sputtering method or a vapor deposition method, since the metal-film forming conditions thereof are easy to control.

The thickness of the metal film is preferably in case of gold: from 5 to 500 nm, in case of silver: from 5 to 500 nm, in case of aluminum: from 5 to 500 nm, in case of copper: from 5 to 500 nm, in case of platinum: from 5 to 500 nm, and in case of alloys thereof: from 5 to 500 nm; and the thickness of a film of chromium is preferably from 1 to 20 nm.

From the standpoint of the electric field enhancement effect, in case of gold: from 20 to 70 nm, in case of silver: from 20 to 70 nm, in case of aluminum: from 10 to 50 nm, in case of copper: from 20 to 70 nm, in case of platinum: from 20 to 70 nm and in case of alloys thereof: from 10 to 70 nm are more preferable; and the thickness of a film of chromium is more preferably from 1 to 3 nm.

If the thickness of the metal film is within the above-described ranges, then surface plasmons will be easily generated, therefore these ranges are suitable. Further, the size (length and width) is not particularly limited, as long as it is a metal film having such a thickness.

### (SAM)

A SAM [Self-Assembled Monolayer] is formed on the other surface of the metal film which surface is not in contact with the transparent support, as a base for immobilizing a ligand and preferably a solid phased layer, or for the purpose of preventing the quenching of a fluorescent molecule by the metal when a plasmon sensor is used in a sandwich assay.

As a monomolecule to be contained by the SAM, usually, carboxyalkanethiol that has a number of carbon atoms of about from 4 to 20 (for example, available from DOJINDO LABORATORIES, Sigma-Aldrich Japan, etc.), especially preferably 10-carboxy-1-decanethiol, is used. Carboxyalkanethiol that has a number of carbon atoms of from 4 to 20 is suitable, since a SAM formed by using it has less optical influence, that is to say, has properties including a high transparency, a low refractive index, a thin film thickness, and so on.

A method for forming such a SAM is not particularly limited, and conventionally known methods can be used. Specific examples thereof include a method in which a transparent support on the surface of which a metal film has been formed, wherein on the film surface a layer composed of a mask material has been formed, is immersed in an ethanol solution containing 10-carboxy-1-decanethiol (manufactured by DOJINDO LABORATORIES), and so on. In this case, a thiol group contained in 10-carboxy-1-decanethiol is bound and immobilized to the metal, and the molecules are self-assembled on the surface of the metal thin film to form a SAM.

Alternatively, before forming a SAM, "a spacer layer composed of a dielectric(s)" may be formed, and, in this case, a monomolecule to be contained by the SAM is not particularly limited, as long as it is a silane coupling agent that has an ethoxy group (or a methoxy group), which can produce a silanol group [Si-OH] by hydrolysis, and has, on the other end, a reactive group, such as an amino group, a glycidyl group, a carboxyl group, or the like; and conventionally known silane coupling agents can be used.

A method for forming such a SAM is not particularly limited, and conventionally known methods can be used.

As a dielectric to be used for forming such "a spacer layer composed of a dielectric(s)", optically transparent inorganic substances, and natural or synthetic polymers may be used. Among these, it is preferable for silicon dioxide [SiO₂], titanium dioxide [TiO₂], or aluminum oxide [Al₂O₃] to be contained, since the chemical stability, the manufacturing stability and the optical transparency thereof are excellent.

The thickness of a spacer layer composed of a dielectric (s) is usually from 10 nm to 1 mm, and, from the standpoint of the resonance angle stability, preferably 30 nm or less, more preferably from 10 to 20 nm. On the other hand, from the standpoint of the electric field enhancement, it is preferably from 200 nm to 1 mm; and, from the standpoint of the stability of the electric field enhancement effect, it is more preferably from 400 nm to 1,600 nm.

Examples of a method for forming a spacer layer composed of a dielectric (s) include, for example, a sputtering method, an electron beam vapor deposition method, a thermal vapor deposition method, a method of forming by a chemical reaction using a material such as polysilazane, or application by using a spin coater, and so on.

### (Solid Phased Layer)

A solid phased layer is formed on the other surface of said SAM which surface is not in contact with said metal film, and has a three dimensional structure.

The term "a three dimensional structure" refers to the structure of a solid phased layer that can expand the immobilization of a ligand as described below not only to the two dimensional area on the surface of a "sensor substrate" (and the vicinity thereof) but also to the three dimensional space that is not in contact with said substrate surface.

Such solid phased layer comprises a polymer composed of at least one monomer selected from the group consisting of glucose, carboxymethylated glucose, and monomers contained in any of vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones, and preferably comprises a hydrophilic polymer such as dextran and dextran derivatives, and a hydrophobic polymer composed of a hydrophobic monomer(s) contained in any of vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones; and dextran such as carboxymethyldextran [CMD] is especially suitable from the standpoint of the bioaffinity, the property of suppressing non-specific adsorption reaction, the high hydrophilicity.

The molecular weight of CMD is preferably from 1 kDa to 5, 000 kDa, more preferably from 4 kDa to 1,000 kDa.

The solid phased layer (for example, a solid phased layer composed of dextran or a dextran derivative) preferably has a density of less than 2 ng/mm². The density of the solid phased layer may be appropriately adjusted according to the type of the polymer to be used. If a polymer as mentioned above within such a density range is solid-phased on a SAM as described above, then, when using a plasmon sensor for an assay method, the assay signal will be stabilized and increased, therefore this range is suitable. The density of that of "Sensor Chip CM5" manufactured by Biacore Life Sciences was 2 ng/mm². This density is a density which was estimated as 2 ng/mm² from the result that measured signals that were obtained by using this CM5 substrate and a substrate whose surface is a gold film only and using a SPR measurement apparatus manufactured by Biacore Life Sciences were determined to be average 2000 RU.

The average film thickness of the solid phased layer is preferably from 3 nm to 80 nm. The film thickness can be measured by using an atomic force microscope [AFM] or the like. If the average film thickness of the solid phased layer is within such a range, then, when using a plasmon sensor for an assay method, the assay signal will be stabilized and increased, therefore this range is suitable.

A method for immobilization onto a SAM surface in case where carboxymethyldextran [CMD] is used as a polymer contained in the solid phased layer will be specifically described.

That is to say, carboxymethyldextran can be immobilized on a SAM by immersing a substrate, on which a transparent support, a metal film and a SAM have been layered in this order, into MES buffered saline [MES], which contains 0.01 mg/mL to 100 mg/mL carboxymethyldextran whose molecular weight is preferably from 1 kDa to 5,000 kDa and which is as described above, 0.01 mM to 300 mM N-hydroxysuccinimide [NHS], and 0.01 mM to 500 mM water-soluble carbodiimide [WSC], for a period of from 0.2 hours to 3.0 hours.

The density of the obtained solid phased layer can be controlled by the number of reaction sites (the number of functional groups of the SAM), the ionic strength and the pH of the reaction solution, and the concentration of WSC to the number of carboxyl groups of the carboxymethyldextran molecule. Further, the average film thickness of a solid phased layer can be controlled by the molecular weight of carboxymethyldextran and the reaction time.

### (Ligand)

In the present invention, the ligand is a molecule or a molecular fragment that can specifically recognize (or be specifically recognized by) and specifically bind to an analyte contained in a sample, and is used for the purpose of immobilizing (capturing) an analyte in a sample when using a plasmon sensor for a sandwich assay.

As such a ligand, a molecule or a molecular fragment that specifically recognizes (or is specifically recognized by) and specifically binds to an analyte, and does not prevent sugar chain recognition by a lectin as a secondary antibody can be used. For example, an antibody or an aptamer that corresponds to a protein, a nucleic acid or the like as an analyte can be used.

For example, in case where the analyte is a glycoprotein, specific examples of an "antibody" which is a ligand thereto include monoclonal antibodies against carcinoembryonic antigens [CEA], such as an anti-α-fetoprotein [AFP] monoclonal antibody (available from Japan Clinical Laboratories, Inc., etc.), an anti-PSA monoclonal antibody, and so on. In addition, for example, an anti-CA19-9 monoclonal antibody (NS19-9), which recognizes a sugar chain moiety as an epitope, can be used as a ligand, as long as said sugar chain moiety does not overlap with a sugar chain moiety that is recognized by the lectin to be used as a secondary antibody.

In the present invention, the term "an antibody (antibodies)" includes polyclonal antibodies or monoclonal antibodies, antibodies obtained by genetic recombination, and antibody fragments.

Examples of a method for immobilization of the ligand include, for example, a method in which a carboxyl group which is contained in a polymer having a reactive functional group such as carboxymethyldextran [CMD] is active esterified by using water-soluble carbodiimide [WSC] (such as 1-ethyl-3-(3-dimethylaminopropyl)carbodiimide hydrochloride [EDC]) and N-hydroxysuccinimide [NHS], and a dehydration reaction between the thus active esterified carboxyl group and an amino group contained in the ligand is carried out using water-soluble carbodiimide to immobilize; a method in which a carboxyl group contained in the SAM as mentioned above is treated as described above, and a dehydration reaction between the resultant carboxyl group and an amino group contained in the ligand is carried out to immobilize, and so on.

Further, in order to prevent non-specific adsorption of a sample and/or the like to the plasmon sensor, it is preferable to treat the surface of the plasmon sensor with a blocking agent, such as bovine serum albumin [BSA], after the immobilization of the ligand as described above.

The density of a ligand immobilized in and outside the solid phased layer is preferably from 1 fmol/cm² to 1 nmol/cm², more preferably from 10 fmol/cm² to 100 pmol/cm². If the density of the ligand is within the above-described ranges, then the signal intensity will be increased, therefore these ranges are suitable.

### <Device for SPFS>

The device for SPFS for use in the present invention is characterized by comprising a plasmon sensor, on one surface of which a ligand is immobilized, wherein an analyte binds to said ligand, and a lectin labeled with a fluorescent dye further binds to the analyte.

Accordingly, the device for SPFS is used for the measurement method of the present invention, and comprises a plasmon sensor on which a ligand, an analyte, and a fluorescently labeled lectin are reacted.

In such a device, for example, a light source of laser light, various kinds of optical filters, a prism, a cut filter, a condenser lens, a fluorescence detection part, and/or the like are comprised in addition to the plasmon sensor, and, in case of using a sample solution, a washing solution, a labeled antibody solution, or the like, a liquid transfer system integrated with the plasmon sensor is preferably contained. As the liquid transfer system, for example, a microchannel device connected to a liquid transfer pump or the like may be used. As a sensor to be used for the detection part, it is preferable to use an image sensor; and a CCD image sensor, a photomultiplier, or the like can be used.

Further, a surface plasmon resonance [SPR] detection part, i.e., a photodiode as a light receiving sensor for SPR, an angle variable part for preparing an angle optimal for SPR and SPFS (wherein, in order to determine the attenuated total reflection [ATR] conditions, by a servomotor, a photodiode and a light source are synchronized and an angle change of from 45° to 85° is allowed; and wherein the resolution is preferably not less than 0.01°), a computer for processing information having been input into the SPFS detection part, and/or the like may also be comprised.

Preferred modes of the light source, the optical filter, the cut filter, the condenser lens and the SPFS detection part are the same as those described above.

Examples of the liquid transfer pump include, for example, a micropump which is suitable for cases where the amount of the transferring liquid is very small; a syringe pump wherein the delivery accuracy is high and the pulsation is small, but it is not possible to circulate; a tube pump which is simple and has an excellent handling property, but may have difficulty in liquid transfer of a very small amount of liquid, and so on.

### EXAMPLES

The present invention will now be described in more detail by way of examples, but the present invention is not limited by these.

### [Example 1] (Detection of Sugar Chain by Lectin Using SPFS)

### (1-1) Fluorescent Labeling of Lectin

AAL [*Aleuria aurantia* Lectin] (SEIKAGAKU CORPORATION) was fluorescently labeled using a commercially available Alexa labeling kit, "Alexa Fluor (trademark) 647 Protein Labeling Kit" (Invitrogen Corporation). The procedures were performed according to the protocol attached to the kit. In order to remove unreacted lectin, unreacted enzyme, and the like, the reaction product was purified using an ultrafiltration membrane (manufactured by Nihon Millipore K.K.) to obtain an Alexa Fluor 647 labeled AAL solution. The obtained fluorescently-labeled lectin solution was stored at 4°C after protein quantification.

### (1-2) Production of Plasmon Sensor

A transparent planar substrate having a thickness of 1 mm and made of glass, "S-LAL10" (manufactured by OHARA INC., Refractive Index [nd] = 1.72), was plasma-cleaned with Plasma Dry Cleaner "PDC200" (manufactured by Yamato Scientific Co., Ltd.). On one side of the plasma-cleaned substrate, first, a chromium film was formed by a sputtering method, and further, on the surface thereof, a gold film was formed by a sputtering method. The thickness of this chromium film was from 1 to 3 nm, and the thickness of the gold film was from 44 to 52 nm.

Then, the thus obtained substrate was immersed for 24 hours in 10 mL of an ethanol solution containing 10-carboxy-1-decanethiol having been adjusted to 25 mg/mL, to form a SAM on the surface of the gold film. This substrate was taken out from the ethanol solution, and washed sequentially with ethanol and isopropanol, and thereafter dried using an air gun.

Subsequently, the substrate on which the SAM has been formed was immersed for 1 hour in PBS that contains 1 mg/mL carboxymethyldextran [CMD] having a molecular weight of 5 hundred thousand, 0.5 mM N-hydroxysuccinimide [NHS], and 1 mM water-soluble carbodiimide [WSC], to immobilize CMD on the SAM. Then, the resultant substrate was immersed in 1N aqueous NaOH solution for 30 minutes to hydrolyze unreacted succinic acid ester.

Onto the surface of the obtained substrate (a surface on which a gold film + a SAM + a solid phased layer having a three dimensional structure are formed in this order), a sheet-shaped silicone rubber spacer having 2 mm x 14 mm hole and having a thickness of 0.5 mm was provided, and the substrate was placed such that said surface was inside the channel (however, such that said silicone rubber spacer was not in contact with the transferring liquid). From the outside of the channel, a polymethyl methacrylate board having a thickness of 2 mm was put on and bonded by pressure so as to cover the substrate, and the channel and said polymethyl methacrylate board were fixed by screws.

As transferring liquids, ultrapure water and then PBS were circulated by a Perista pump at room temperature at a flow rate of 500 µL/min for 10 minutes and for 20 minutes, respectively. Subsequently, 5 mL PBS containing 50 mM NHS and 100 mM WSC was sent and circulation liquid transfer was performed for 20 minutes. Thereafter, circulation liquid transfer of 2.5 mL of an anti-α-fetoprotein [AFP] monoclonal antibody (1D5; 2.5 mg/mL; manufactured by Japan Clinical Laboratories, Inc.) solution was performed for 30 minutes. Thereby, the primary antibody was solid-phased on the solid phased layer having a three dimensional structure. Finally, a treatment for preventing non-specific adsorption was carried out by performing circulation liquid transfer of PBS buffered saline containing 1% by weight of bovine serum albumin [BSA] for 30 minutes. Thereby, a plasmon sensor was produced.

### (1-3) Real-Time Measurement

Step (a): The transferring liquid was switched to PBS, and 0.5 mL (500 µL) of each solution obtained by carrying out serial dilution to the AFP concentrations of 10, 2, 0.5, and 0.1 ng/mL was added, followed by circulation thereof for 25 minutes.

Washing Step: Washing was carried out by circulating TBS containing 0.05% by weight of Tween 20 as a transferring liquid for 10 minutes. Here, the fluorescence of a blank was detected as follows: LD laser was used as a light source; after the photon amount was adjusted by a wavelength-selective polarizing filter, laser light having a wavelength of 635 nm was irradiated, through a prism (manufactured by OHARA INC., "S-LAL10" (Refractive Index [n] = 1.72)), to the plasmon sensor fixed in the channel; and, using as a cut filter a cut filter that cuts light having the wavelengths other than the wavelength of light of the fluorescence component, and using as a condenser lens a 20 x objective lens, detection by a CCD image sensor was carried out.

Step (b): 5 mL PBS containing 1,000 ng/mL of the fluorescently labeled lectin obtained in (1-1) was added, followed by circulation thereof for 20 minutes. From immediately after the addition of the solution, measurement of the fluorescence intensity was carried out, and the liquid transfer time and the signals detected by a CCD were plotted. The results of the real-time measurement are shown in Fig. 1.

Washing Step: The transferring liquid was switched from the fluorescently labeled lectin solution to a TBS solution containing 0.05% by weight of Tween 20, and, for 20 minutes from immediately after the switching, the dissociation reaction of the fluorescently labeled lectin from the antigen was observed by measuring the fluorescence signals by a CCD. The plotted results of signals after washing are shown in Fig. 2.

### [Comparative Example 1] (ELISA)

### (2-1) Biotin Labeling of Lectin

AAL [*Aleuria aurantia* Lectin] (SEIKAGAKU CORPORATION) was biotin-labeled using a commercially available biotin labeling kit ("Biotin Labeling Kit - NH2", DOJINDO LABORATORIES). The procedures were performed according to the protocol attached to the kit. In order to remove unreacted biotinylation reagent, the reaction product was purified using an ultrafiltration membrane (manufactured by Nihon Millipore K.K.) to obtain a biotin-labeled AAL solution. The obtained solution was stored at 4°C after protein quantification.

### (2-2) Production of ELISA Plate

To a 96-well plate, an anti-AFP antibody (clone: 1D5) having been adjusted to 5 µg/L was aliquoted in 50 µL volumes, and immobilization was carried out at 4°C overnight. Thereafter, the antibody solution was removed from the 96-well plate, and 1% PBS-BSA (-) was aliquoted thereto in 100 µL volumes, and immobilization was carried out at 4°C overnight.

### (2-3) Assay

The solution of (2-2) was removed, and thereafter AFP antigen of an optional concentration (0.1, 0.5, 2, 10, 50, 100, 200 ng/mL) was aliquoted thereto in 50 µL volumes, followed by shaking the plate at 37 °C for 1 hour. The solution was discarded, and the plate was washed with 200 µL PBS containing Tween 20. Thereafter, a biotin-labeled lectin having been adjusted to 10 µg/mL was added thereto, and the plate was shaken at 37°C for 1 hour. The solution was discarded, and the plate was washed with 200 µL PBS containing Tween 20. Thereafter, 0.125 µg/mL streptavidin-HRP was aliquoted thereto in 50 µL volumes, and the plate was shaken at 37°C for 30 minutes. The solution was discarded, and the plate was washed with 200 µL PBS containing Tween 20. Thereafter, light emission was carried out by SuperSignal ELISA Femto Maximum Sensitivity Substrate (manufactured by Thermo scientific), and measured by a plate reader Fluoroskan Ascent (Thermo Fisher Scientific, Inc. (America)). The obtained results are shown in Fig. 2.

### Industrial Applicability

The method for measuring the amount of an analyte of the present invention is a method by which an analyte can be detected with a high sensitivity and a high accuracy. Therefore, for example, even an extremely small amount of tumor marker contained in blood can be detected, and, from the results, the presence of preclinical non-invasive cancer (cancer in situ) which is undetectable by palpation and/or the like can also be predicted with a high accuracy.

## Claims

1. A method for measuring the amount of an analyte in a sandwich assay using a surface plasmon excitation enhanced fluorescence spectroscopy [SPFS], wherein the sandwich assay uses a solid phase primary antibody immobilized in and outside a solid phased layer having a three dimensional structure and a lectin labeled with a fluorescent dye is used as a secondary antibody, wherein said solid phased layer comprises a polymer composed of at least one monomer selected from the group consisting of glucose; carboxymethylated glucose; and monomers contained in any of vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones; and wherein the reaction between said lectin and said analyte is carried out simultaneously with measurement of the amount of the fluorescence by the SPFS.

2. The measurement method according to claim 1, in which said assay is carried out within a channel, and the flow rate of the transferring liquid is from 100 µL/min to 10,000 µL/min.

3. The measurement method according to claim 1 or 2, in which said analyte is a tumor marker.

4. The measurement method according to any one of claims 2 to 3, in which the amount of a sample to be supplied to said channel is from 5 µL to 1,000 µL.

5. Use of a plasmon sensor for carrying out the method of one of claims 1 to 4, wherein on one surface of the sensor a ligand is immobilized, wherein said ligand is an antibody immobilized in and outside a solid phased layer having a three dimensional structure, wherein an analyte binds to said ligand, and a lectin labeled with a fluorescent dye further binds to said analyte, and wherein said solid phased layer comprises a polymer composed of at least one monomer selected from the group consisting of glucose; carboxymethylated glucose; and monomers contained in any of vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones.

6. The use according to claim 5, in which said analyte is a tumor marker.

## Patentansprüche

1. Ein Verfahren zum Messen der Menge eines Analyten in einem Sandwich-Test unter Verwendung einer Oberflächenplasmonen-anregungsverstärkten Fluoreszenzspektroskopie [SPFS], wobei der Sandwich-Test einen Festphasenprimärantikörper verwendet, der in und außerhalb einer Festphasenschicht mit einer dreidimensionalen Struktur immobilisiert ist und ein mit einem Fluoreszenzfarbstoff markiertes Lektin als Sekundärantikörper verwendet wird, wobei besagte Festphasenschicht ein Polymer umfasst, das aus mindestens einem Monomer zusammengesetzt ist, ausgewählt aus der Gruppe bestehend aus Glukose; carboxymethylierter Glucose; und Monomeren, die in einem aus Vinylestern, Acrylsäureestern, Methacrylsäureestern, Olefinen, Styrolen, Crotonsäureestern, Itaconsäurediestern, Maleinsäurediestern, Fumarsäurediestern, Allylverbindungen, Vinylethern und Vinylketonen enthalten sind; und wobei die Reaktion zwischen besagtem Lektin und besagtem Analyten gleichzeitig mit der Messung der Fluoreszenzmenge durch die SPFS durchgeführt wird.

2. Die Messmethode nach Anspruch 1, worin besagter Test innerhalb eines Kanals durchgeführt wird und die Durchflussrate der übertragenen Flüssigkeit von 100 µL/min bis 10.000 µL/min beträgt.

3. Die Messmethode nach Anspruch 1 oder 2, worin besagter Analyt ein Tumormarker ist.

4. Die Messmethode nach einem der Ansprüche 2 oder 3, worin die Menge einer Probe, die besagtem Kanal zugeführt wird, 5 µL bis 1.000 µL ist.

5. Verwendung eines Plasmonen-Sensors zur Durchführung des Verfahrens nach einem der Ansprüche 1 bis 4, wobei auf einer Oberfläche des Sensors ein Ligand immobilisiert ist, wobei besagter Ligand ein Antikörper ist, der in und außerhalb einer Festphasenschicht mit einer dreidimensionalen Struktur immobilisiert ist, wobei ein Analyt an besagten Liganden bindet und ein mit einem Fluoreszenzfarbstoff markiertes Lektin weiter an besagten Analyten bindet, und wobei besagte Festphasenschicht ein Polymer umfasst, das aus mindestens einem Monomer besteht, ausgewählt aus der Gruppe bestehend aus Glukose; carboxymethylierter Glukose; und Monomeren, die in einem aus Vinylestern, Acrylsäureestern, Methacrylsäureestern, Olefinen, Styrolen, Crotonsäureestern, Itaconsäurediestern, Maleinsäurediestern, Fumarsäurediestern, Allylverbindungen, Vinylethern und Vinylketonen enthalten sind.

6. Die Verwendung nach Anspruch 5, worin besagter Analyt ein Tumormarker ist.

## Revendications

1. Procédé de mesure de la quantité d'une substance à analyser dans un essai de sandwich en utilisant la spectroscopie de fluorescence renforcée par l'excitation de plasmons de surface [SPFS], dans lequel l'essai de sandwich utilise un anticorps primaire en phase solide immobilisé à l'intérieur et à l'extérieur d'une couche en phase solide ayant une structure tridimensionnelle et dans lequel une lectine marquée avec un colorant fluorescent est utilisée comme un anticorps secondaire, dans lequel ladite couche en phase solide comprend un polymère composé d'au moins un monomère sélectionné dans le groupe composé de glucose, de glucose carboxyméthylé; et de monomères contenus dans des esters vinyliques, des esters d'acide acrylique, des esters d'acide méthacrylique, des oléfines, des styrènes, des esters d'acide crotonique, des diesters d'acide itaconique, des diesters d'acide maléique, des diesters d'acide fumarique, des composés allyliques, des esters vinyliques et des vinylcétones, et dans lequel la réaction entre ladite lectine et ladite substance à analyser s'effectue simultanément avec la mesure de la quantité de la fluorescence par moyen de SPFS.

2. Procédé de mesure selon la revendication 1, dans lequel ledit essai est effectué à l'intérieur d'un canal et le débit du liquide circulant est compris entre 100 µl/min et 10.000 µl/min.

3. Procédé de mesure selon la revendication 1 ou la revendication 2, dans lequel ladite substance à analyser est un marqueur de tumeur.

4. Procédé de mesure selon l'une quelconque des revendications 2 à 3, dans lequel la quantité d'un échantillon à être amené audit canal est comprise entre 5 µl et 1.000 µl.

5. Utilisation d'un capteur de plasmons pour effectuer le procédé selon l'une des revendications 1 à 4, dans lequel un ligand est immobilisé sur une surface du capteur, dans lequel ledit ligand est un anticorps immobilisé à l'intérieur et à l'extérieur d'une couche en phase solide ayant une structure tridimensionnelle et dans lequel une substance à analyser se lie audit ligand, et une lectine marquée avec un colorant fluorescent se lie en outre à ladite substance à analyser, et dans lequel ladite couche en phase solide comprend un polymère composé d'au moins un monomère sélectionné dans le groupe composé de glucose, de glucose carboxyméthylé ; et de monomères contenus dans des esters vinyliques, des esters d'acide acrylique, des esters d'acide méthacrylique, des oléfines, des styrènes, des esters d'acide crotonique, des diesters d'acide itaconique, des diesters d'acide maléique, des diesters d'acide fumarique, des composés allyliques, des esters vinyliques et des vinylcétones.

6. Utilisation selon la revendication 5, dans lequel ladite substance à analyser est un marqueur de tumeur.
